**Europäisches Patentamt**

**European Patent Office**

**Office européen des brevets**

(19)

(11) Veröffentlichungsnummer : **0 302 003 B1**

(12)

# EUROPÄISCHE PATENTSCHRIFT

(45) Veröffentlichungstag der Patentschrift :
**22.04.92 Patentblatt 92/17**

(51) Int. Cl.$^5$ : **G01N 1/28,** G01N 1/04,
G01N 33/20, G01N 29/04

(21) Anmeldenummer : **88730154.7**

(22) Anmeldetag : **07.07.88**

(54) **Verfahren zur Herstellung von Proben für die Reinheitsgradbestimmung von stranggegossenem Stahl mittels Ultraschall.**

(30) Priorität : **22.07.87 DE 3724627
23.10.87 DE 3736389**

(43) Veröffentlichungstag der Anmeldung :
**01.02.89 Patentblatt 89/05**

(45) Bekanntmachung des Hinweises auf die
Patenterteilung :
**22.04.92 Patentblatt 92/17**

(84) Benannte Vertragsstaaten :
**AT BE CH FR GB IT LI**

(56) Entgegenhaltungen :
**EP-A- 0 189 554
AT-B- 340 178
DE-B- 2 436 983
GB-A- 1 046 443**

(73) Patentinhaber : **MANNESMANN
Aktiengesellschaft
Mannesmannufer 2
W-4000 Düsseldorf 1 (DE)**

(72) Erfinder : **Gronsfeld, Josef, Dr.-Ing.
Sternstrasse 6
W-4154 Tönisvorst 1 (DE)**
Erfinder : **Jacobi, Hatto, Dr.-Ing.
Rosspfad 42
W-4000 Düsseldorf 31 (DE)**
Erfinder : **Bäthmann, Hans-Jürgen
Höhenweg 49
W-4130 Moers 2 (DE)**

(74) Vertreter : **Meissner, Peter E., Dipl.-Ing. et al
Patentanwaltsbüro Meissner & Meissner,
Herbertstrasse 22
W-1000 Berlin 33 (DE)**

EP 0 302 003 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Proben für die Reinheitsgradbestimmung von Metallen.

Unter Metallen werden Eisen- und Nichteisenmetalle sowie deren Legierungen verstanden, die infolge ihres metallurgischen Herstellungsprozesses nicht gewünschte Verunreinigungen enthalten. Diese Verunreinigungen bestehen im wesentlichen aus nichtmetallischen Phasen, die temperaturabhängig verformbar sind. Durch eine Untersuchung mittels Ultraschall und entsprechender Anzeige kann der im Metall entahltene Anteil erkannt und ausgewertet werden. Diese Untersuchungen werden im Rahmen der Reinheitsgradbestimmungen durchgeführt. Im Bereich der Eisenlegierungen findet die Erfindung vorzugsweise Anwendung bei Stählen, die im Stranggußverfahren vergossen wurden.

Mit steigenden Anforderungen an das Produkt wird die Bestimmung des Reinheitsgrades problematischer. Standardisierte Prüfmethoden büßen zunehmend an Aussagekraft ein. Mehr und mehr wird die Ausfallrate am Produkt - bei der Fertigung oder im Gebrauch - als Kennwert für den Reinheitsgrad herangezogen. Für die Stahlherstellung und metallurgische Entwicklungen ist diese Situation unbefriedigend, weil keine Rückkoppelung zum Verbraucher besteht oder die Information viel zu spät kommt. Weltweit besteht deshalb ein Bedarf an neuen Methoden, die rationell, schnell, kostengünstig, zuverlässig, quantifizierbar und differenzierend auf einzelne Abschnitte eines Erzeugnisses, insbesondere Strangabschnitte, anwendbar sind. Mit der hier geschilderten neuen Methode wird dieses Ziel erreicht.

Bekannte Prüfmethoden auf Reinheitsgrad

– Schwefelabzug (Baumannabdruck)
– Metallografie nach Stahleisenprüfblatt 1570
– Stufendrehprobe nach Stahleisenprüfblatt 1580
– Blaubruchproben
– Ultraschall am Grobblech, Ganztafel oder Randzonenprüfung
– Slime Extraction Methode (Rückstandsisolierung)
– Quantitative Metallografie an 200 cm² großen Flächen

Eine Kritik dieser Prüfmethoden würde hier den Rahmen sprengen. Aus der Sicht der Reinheitsgradentwicklung werden die bekannten Methoden der Zielsetzung nicht gerecht. Das Fehlen eines geeigneten Instrumentariums ließ seit vielen Jahren praktisch keine Beurteilung über Erfolg oder Mißerfolg von Maßnahmen im Stahlwerk zu.

Der Erfindung liegt die Aufgabe zugrunde, ein Verfahren zur Herstellung von Proben zu schaffen, mit dem es möglich ist, Ultraschall-Untersuchungen durchzuführen, die eine exakte Reinheitsgradbestimmung bezogen auf das Erzeugnis selbst zulassen.

Gelöst wird diese Aufgabe mit den im Anspruch 1 angegebenen Merkmalen.

Vorteilhafte Weiterentwicklungen der Erfindung sind in den abhängigen Ansprüchen enthalten.

Kerngedanke der Erfindung ist eine Konzentrierung der Einschlüsse bzw. Verunreinigungen in bestimmten Bereichen, so daß aus der dort angetroffenen Anhäufung eine Aussage über den Reinheitsgrad eines Erzeugnisses, insbesondere des Stranggußmaterials selbst möglich ist.

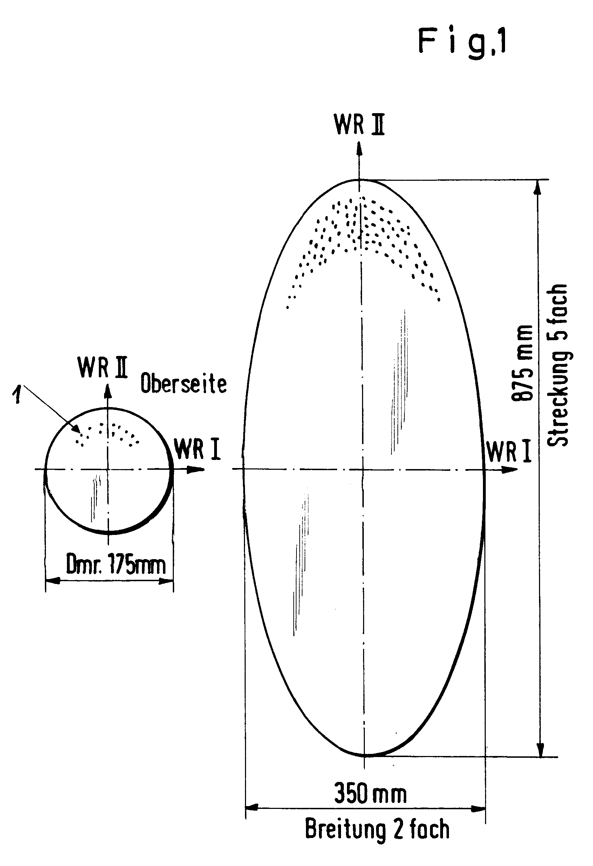

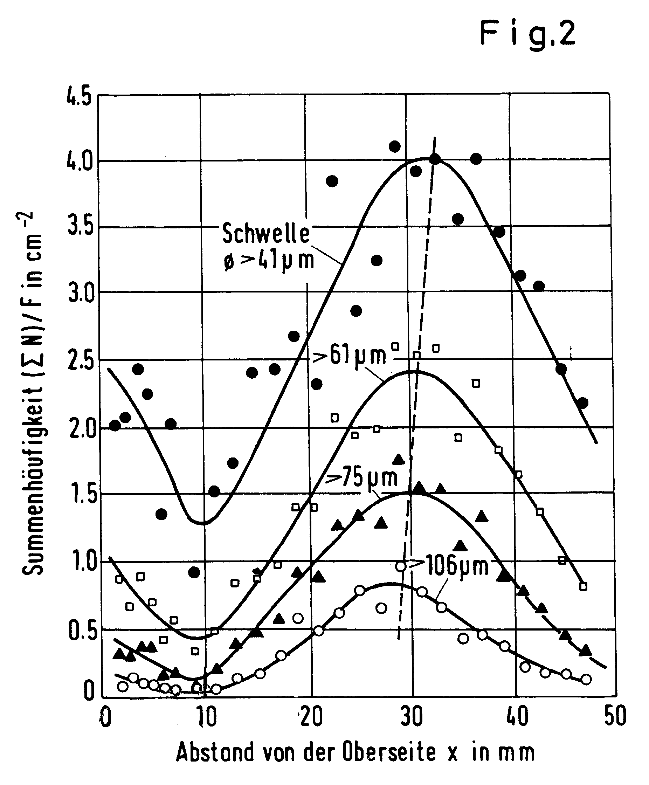

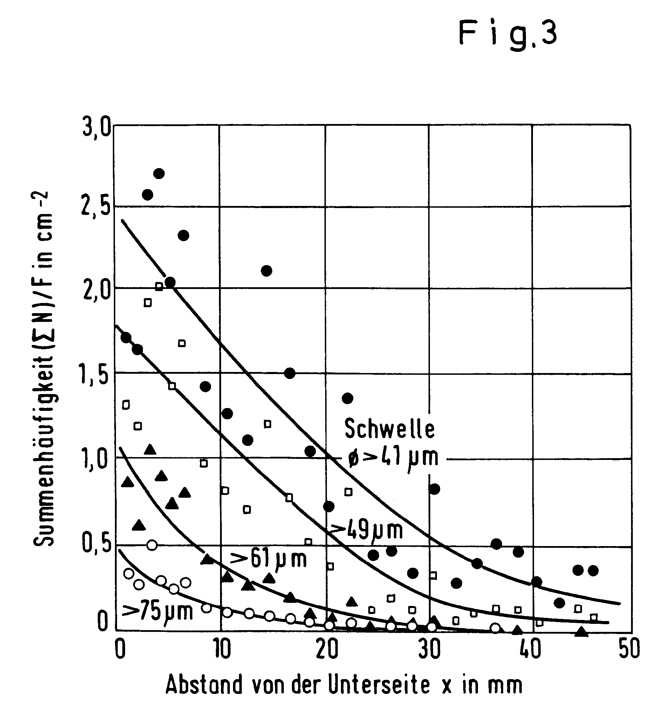

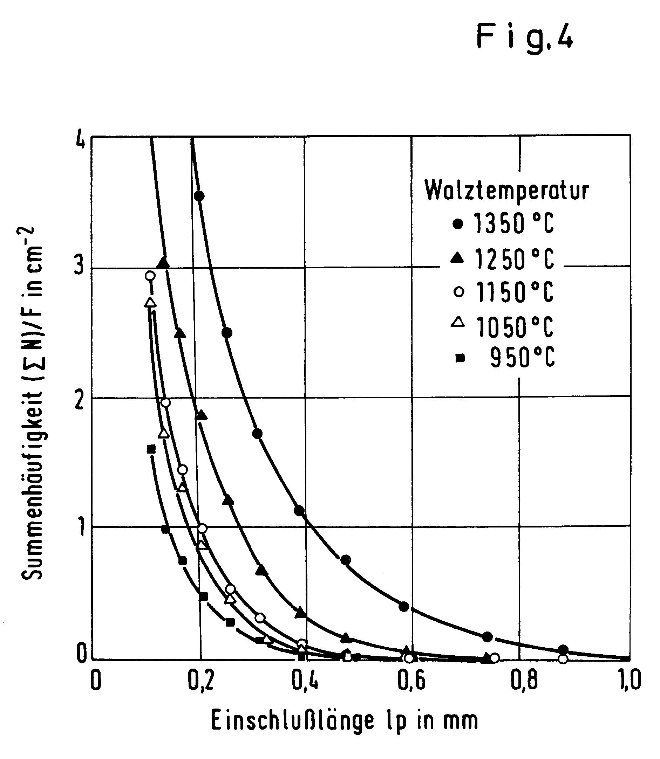

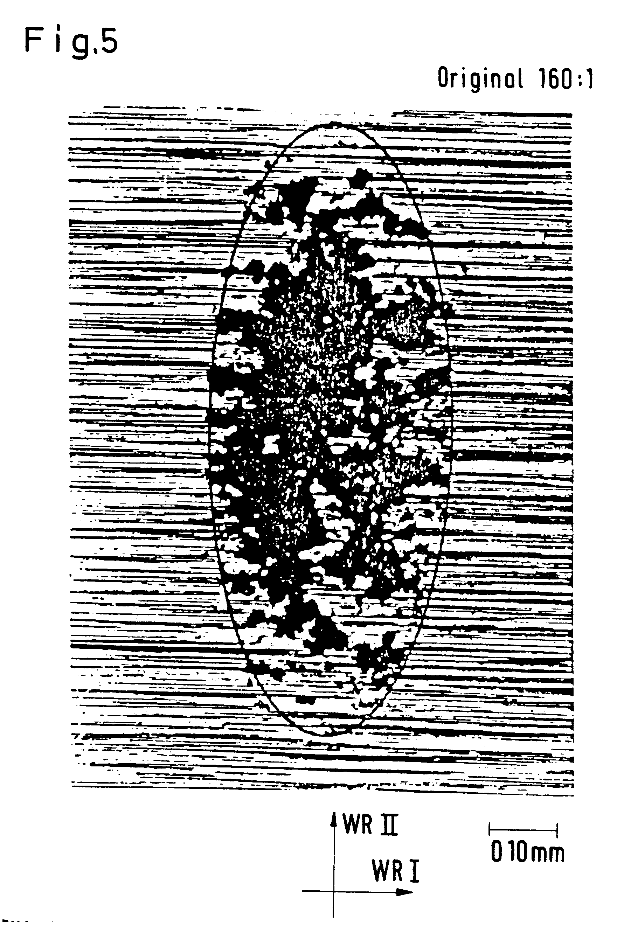

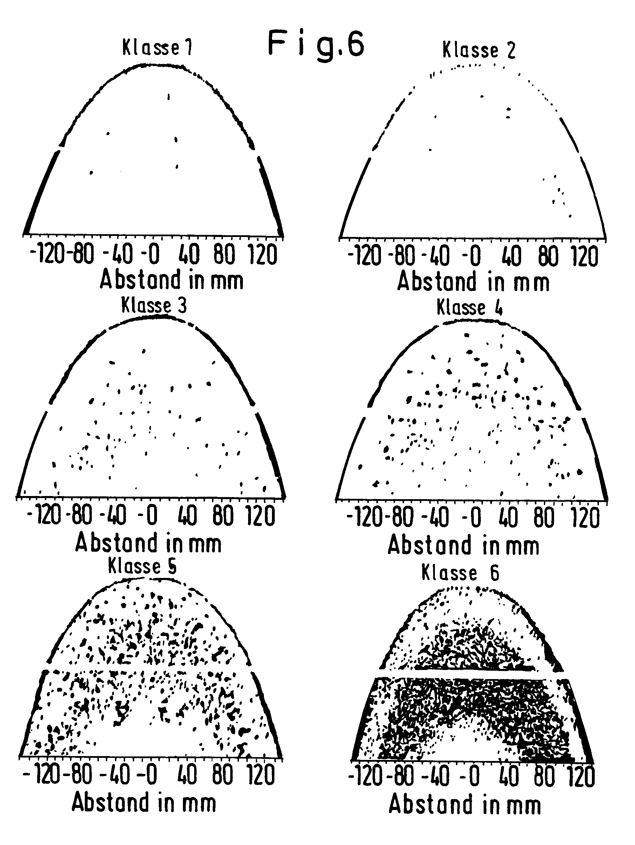

Erläutert werden soll dieses Verfahren anhand der Zeichnungen, und zwar der Figuren 1 bis 12.

Die Erfindung soll zunächst anhand eines Beispieles, das sich auf einen Rundstrang aus Stahl bezieht, erläutert werden. Hierbei werden alle Einschlüsse 1 einer 25 kg Rundstrangprobe auf ein relativ kleines Ultraschall-Prüfblech konzentriert. Wie in Figur 1 veranschaulicht, wird ein Probenrohling in Form eines Zylinders (Hôhe 120 mm) in ein "Surfbrett" ausgewalzt Bleckducke 12 zum; Dickenreduziezrung zehn fach. Die Walzrichtung ist senkrecht zur Achse des Rundstranges. Von der Unterseite zur Oberseite des Bogenstranges wird gestreckt (fünffach; Walzrichtung II), quer dazu wird gebreitet (zweifach; WR I). Das Einschlußband wird gestaucht. Die hohe Teilchendichte pro Volumeneinheit bleibt erhalten. Im Endeffekt werden die sauberen und schmutzigen Zonen des Stranges für die US-Prüfung säuberlich getrennt. Ballast für die Prüfung wird abgeworfen. Die Einschlüsse werden gestreckt und gebreitet und auf diese Weise für die US-Prüfung sensibilisiert. Trotz einer hinreichenden Umformung sinkt die Dicke der Probe (Blechdicke) nicht unter einen für den Ultraschall kritischen Wert.

Die Erfindung basiert auf grundlegenden Untersuchungen zur Lage des Einschlußbandes in Bogensträngen.

Figur 2 zeigt das Größenspektrum der Einschlüsse auf der Oberseite eines verunreinigten 177-mm- Rundstranges.

In Figur 3 ist demgegenüber die saubere Unterseite desselben Stranges dargestellt. Aufgrund von Untersuchungen der Erfinder zur Verformbarkeit der Einschlüsse im Stahl ist für die Ziele der Erfindung eine möglichst hohe Walztemperatur, z.B. 1350 °C, notwendig.

Figur 4 Längenversteilung dergestreckten Eindchlüsse zeigt, daß die Einschlüsse von ursprünglich kugeliger Gestalt mit zunehmender Walztemperatur stärker gestreckt werden.

In Figur 5 ist ein elliptisch ausgewalzter Einschluß parallel zur Walzebene ausgebildet. Der Einschluß ist zertrümmert, aber die elliptische Kontur ist erkennbar.

Figur 6 zeigt einige Beispiele von US-geprüften "Surfbrettproben", die hier in einem Klassensystem geordnet sind.

| Klasse | Anzeigenhdufigkeit |
|---|---|
| 0 | Keine Auzeigen |
| 1 | vereinzette Anzeigen:<10 Anzeigen |
| 2 | wenige Anzeigen:<20 Anzeigen |
| 3 | erkennbares Einschlußband |
| 4 | ausgeprägtes Einschlußband |
| 5 | sehr dichtes Einschlußband |
| 6 | extreme Anzeigendichte, z.B. durch abgenissenen Tauchausguß hervorgerufen. |

Die Beispiele sind real und metallurgisch begründbar. Als Vergleich sei angemerkt, daß der Baumannabdruck zwischen diesen Fällen nicht differenziert. Im konkreten Fall der Probe mit Klasse 6 war der S-Abzug sogar weiß, weil emulgierte Gießschlacke offenbar vom S-Abzug nicht angezeigt wird.

Die Abwicklung des Prüfprogrammes sieht folgendermaßen aus: Rundstrangproben werden planparallel auf 130 mm Länge in Gießrichtung gesägt oder gebrannt. Die Strangoberseite wird anhand der Anpreßbahnen der Stützrollen identifiziert. Dann wird die Unterseite ca. 10 mm tief eingesägt. Der Schlitz dient der Orientierung beim Walzen. Die Rundproben werden auf 1350 °C aufgeheizt. In WR I wird nachfolgendem Stichplan gebreitet: 130 - 120 - 105 - 90 - 80 - 70 - 60 mm. Dann wird die nunmehr elliptische Probe um 90° gedreht und in folgenden Stichen gestreckt (WR II): 48 - 36 - 18 - 13 mm. An der Lage der Kerbe läßt sich ablesen, wie gut symmetrisch gewalzt wurde. Die elliptischen "Surfbretter" werden durch Sägen zerteilt. Auf der Strangeoberseite werden zwei 100 mm breite Streifen vom Scheitel der Ellipse abgetrennt, auf der Unterseite genügt ein Streifen zur Kontrolle, ob nicht eventuell Ober- und Unterseite vor der Walzung verwechselt wurden. Die drei Probenstreifen werden planparallel geschliffen und auf einer HIC-Prüfapparatur geprüft.

Bei Bogenstranggießanlagen lagern sich die Einschlüsse grundsätzlich im Bereich der Strangoberseite ab. Dies gilt erst recht für den Horizontalstrangguß. Dieses Phänomen ist unabhängig vom Strangformat.

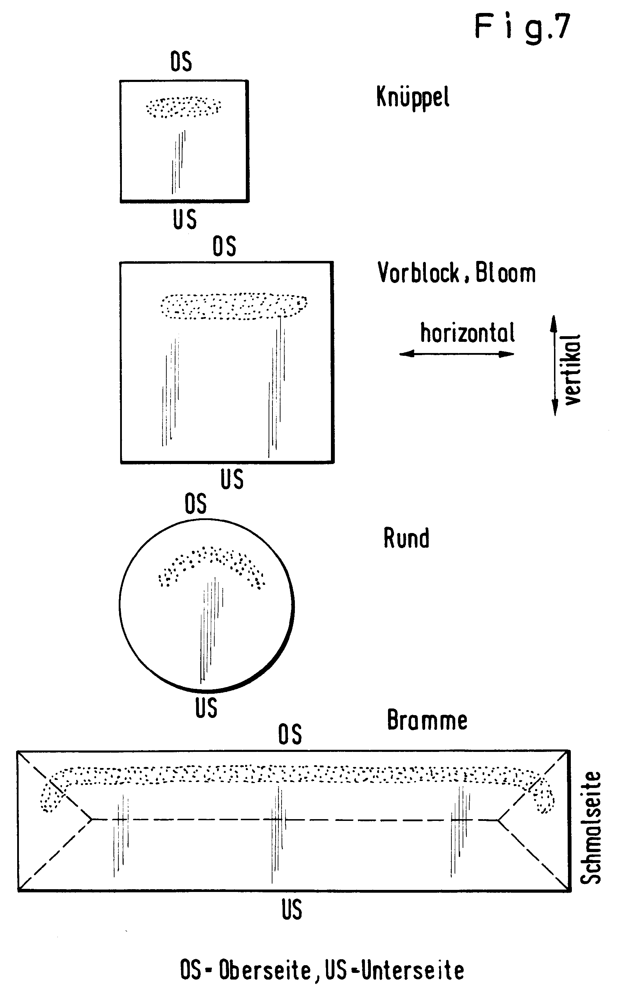

Figur 7 zeigt schematisch die Lage des Einschlußbandes bei Knüppeln, Blooms und Brammen auf Schmitten senkrecht zur Gießrichtung. Durch eine geeignete Walzung von bestimmten Strangabschnitten mit Stauchung des Einschlußbandes kann ein geeignetes Prüfblech erzeugt werden, in dem die Einschlüsse konzentriert und sensibilisiert vorliegen. Die Stauchrichtung kann parallel zur Gießrichtung sein. Sie kann aber auch, bezogen auf die Strangorientierung in der Gießanlage, horizontal sein, d.h. quer zum Gußprodukt. An den Schmalseiten der Bramme kann die Stauchrichtung vertikal sein, obwohl eine solche Walzung, die der herkömmlichen entspräche, nicht den größten Effekt verspricht, sondern allenfalls Resultate wie bei der herkömmlichen Randzonenprüfung.

Aus dem Gußprodukt können passende Walzblöcke herausgesägt werden, wobei allerdings eine 10fache Dickenreduzierung zur Sensibilisierung der Einschlüsse notwendig ist. Außerdem darf das Prüfblech nicht dünner als 10 mm werden, weil der Ultraschall die ersten Millimeter Dicke eines Prüfbleches, d.h. den oberflächennahen Bereich nicht erfaßt. In bezug auf die Ausgangsdicke des Walzblockes gibt es keine prinzipielle Grenze, sie kann aber von den örtlichen Walzmöglichkeiten abhängen.

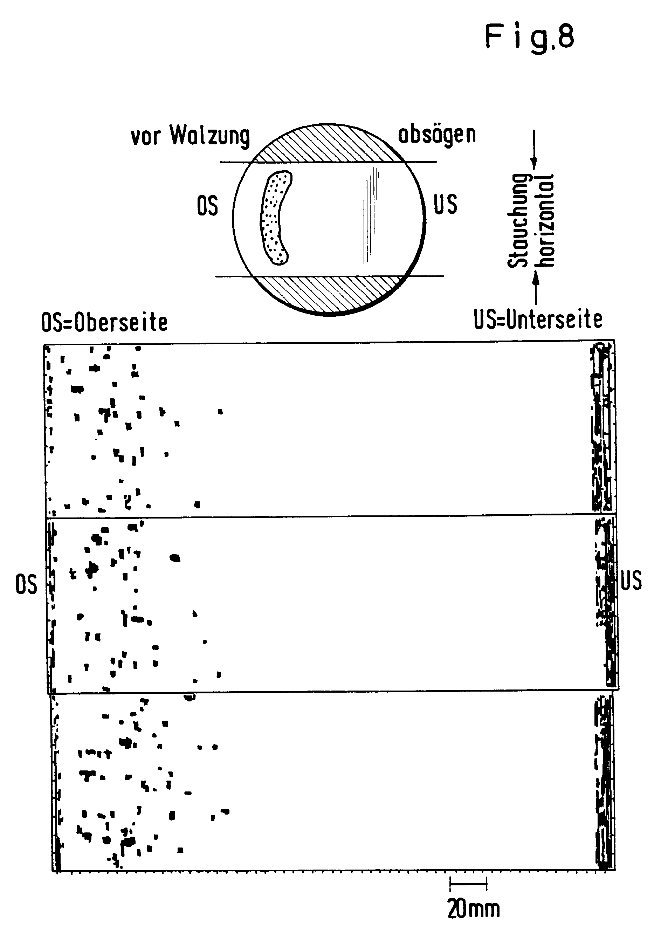

In Figur 8 ist für die Rundstränge die horizontale Stauchung des Einschlußbandes durch Walzen parallel und senkrecht zur Gießrichtung erklärt. Vor dem Walzen werden Segmente des Rundquerschnittes abgesägt, damit das Walzgut glatt aufliegt und kontrolliert in den Walzspalt geführt werden kann. Der US-Befund in Figur 8 bestätigt die Anwendbarkeit dieser Art der Walzung. Wenn das vorhergehende Sägen ungenau ist, d.h. nicht im Winkel von 90° zum Einschlußband, dann reichen die US-Anzeigen bis an den Blechrand.

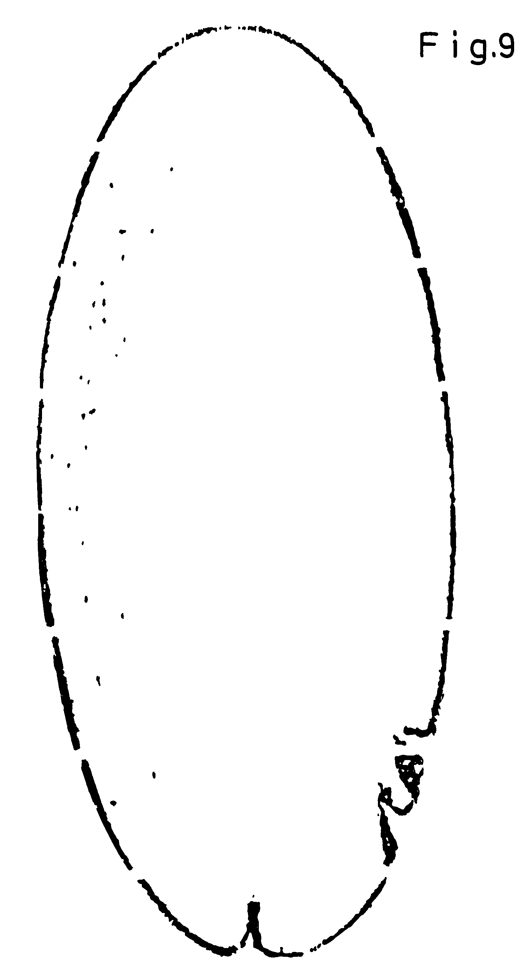

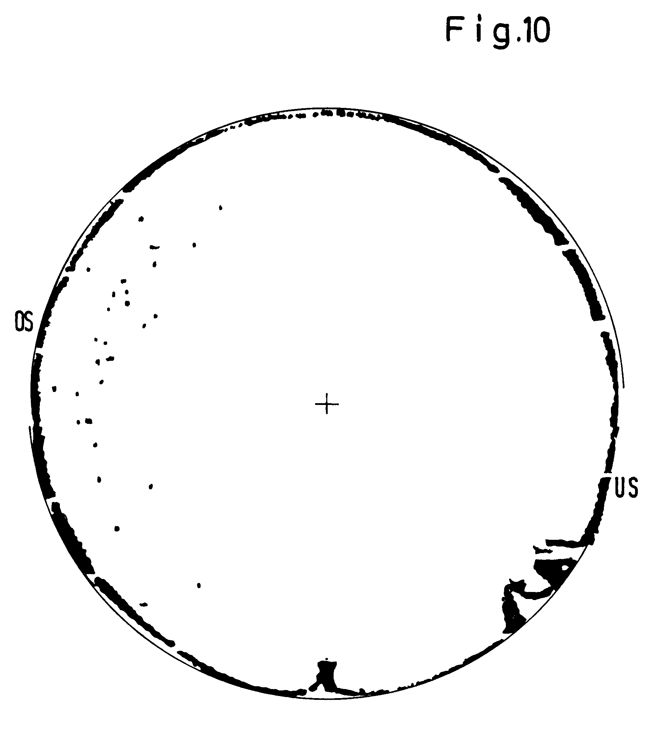

Die Erfindung kann benutzt werden, um stranggegossenes Vormaterial unbekannter Herkunft bezüglich Gießoberseite und -unterseite zu orientieren und den Reinheitsgrad des Materials zu untersuchen. Ein Vormateriallieferant möge einen nach dem Bogenstranggießverfahren hergestellten Strang im Format 360 mm x 420 mm erzeugen und diesen zu 177 mm Röhrenrundmaterial walzen. Beizscheibe und S-Abzug liefern keine Information. Das 177 mm-Rundmaterial wird willkürlich an zwei Stellen des Mantels gekerbt (und zum Surfbrett gewalzt). Figur 9 zeigt das US-Prüfergebnis. Von der Ellipse wird mathematisch auf das Rundmaterial rücktransformiert, s. Figur 10. Jetzt wird die Richtung Gießoberseite-Gießunterseite festgelegt. Weitere 177 mm-Rundproben können mit Kenntnis der Orientierung gezielt auf den Reinheitsgrad untersucht werden. Auf diese Weise kann man auch im Nachhinein Blockguß vom Bogenstrangguß unterscheiden, was nach Vorverformung normalerweise sehr schwierig ist.

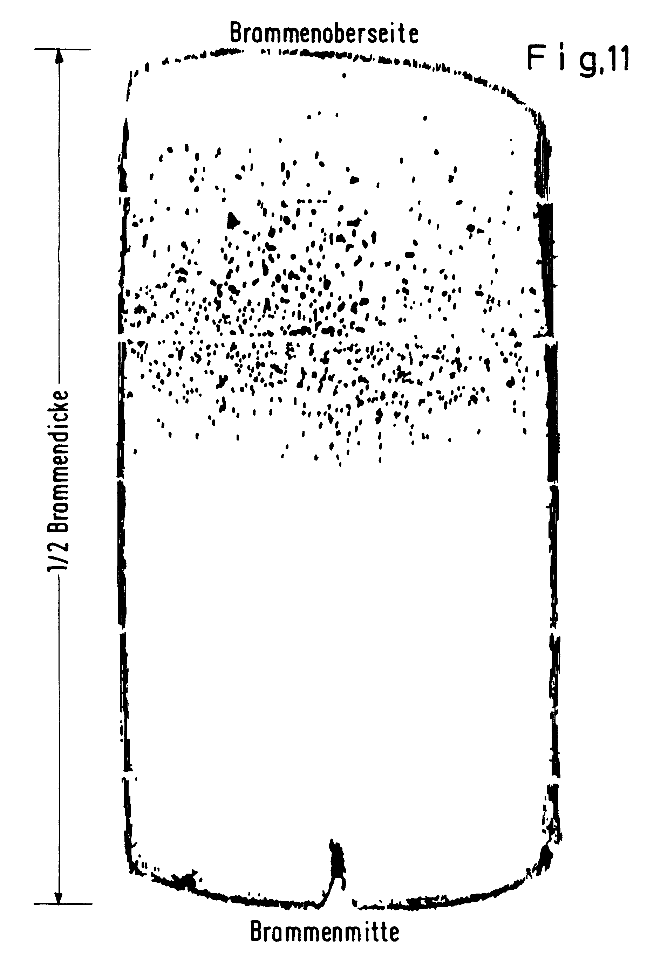

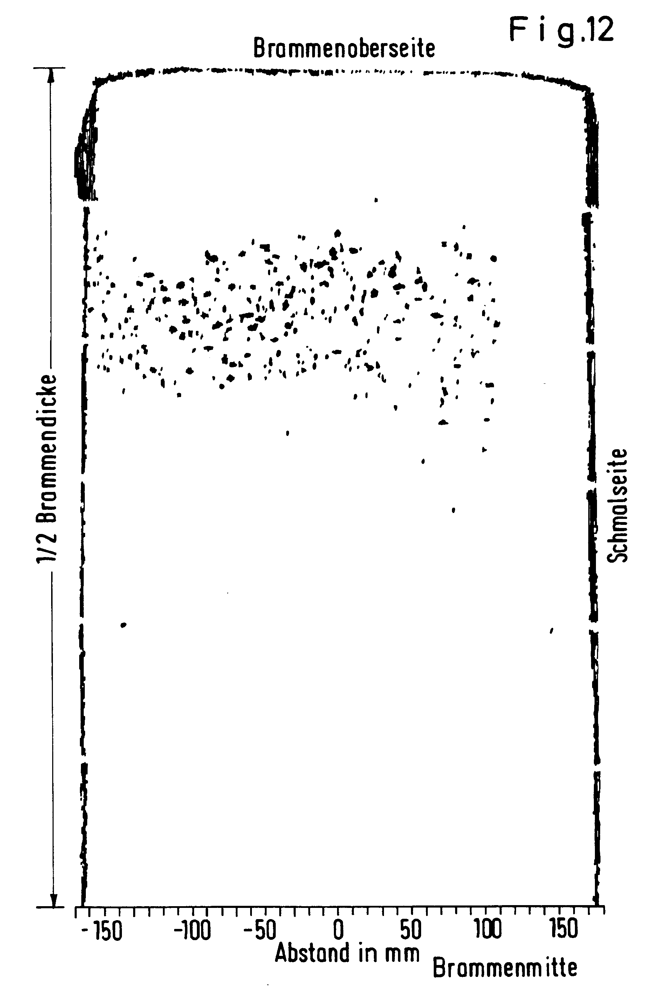

Figur 11 zeigt eine Anwendung der Erfindung auf eine Stranggußbramme. Der S-Abzug hatte keinen übermäßig schlechten Reinheitsgrad angezeigt. Dargestellt ist das US-Ergebnis in der oberen Brammenhälfte. Die untere Hälfte war frei von Anzeigen. Die analysierte Brammenprobe lag bei 1/2 Brammenbreite. In Figur 12 wurde an einer ganz anderen Bramme der Bereich der Schmalseite geprüft. Dort tritt eine Speckschicht auf, die frei von Einschlüssen ist. An beiden gezeigten Brammenproben wurde wie bei der Standardwalzung von Rundmaterial zweifach gebreitet und fünffach gestreckt. Für die genaue Ermittlung der Abstände und Dicken von Speckshicht und Einschlußband müßte vom Blech auf das Gußprodukt rücktransformiert werden. Das ist hier jedoch nicht geschehen. Fig.12 zeigt das Ultraschallergebnis an der gewalzten Brammenprobe (obere Hälfte) aus dem Bereich der Schmalseite.

Die Erfindung ist auch anwendbar auf "dünnwandige" Produkte, wie z.B. Rohre oder Bleche oder auch auf Querschnitte, z.B. unter 100 mm Durchmesser oder Kantenlänge, die an sich aufgrund ihres Schlankheitsgrades nicht in der Längserstreckung der Probe stauchbar sind (Knicken der Probe unter Druck).

Bei diesen Produkten kann derart verfahren werden, daß z.B.

a) mehrere Blechproben gebündelt und mechanisch zusammengehalten werden,

b) bei rohrförmigen Probenabschnitten eine entsprechender Kern oder

c) bei Stäben eine entsprechende rohrförmige Hülse Anwendung findet.

Durch diese Maßnahmen wird ein die Verformung ohne Knickung ermöglichender Körper hergestellt, wobei die Hilfsmittel mitverformt und anschließend abgetrennt werden.

## Patentansprüche

1. Verfahren zur Herstellung von Proben für die Ermittlung von Verunreinigungen in Metallen und Stählen, insbesondere von im Stranggießverfahren vergossenen Stählen, dadurch gekennzeichnet, daß für die Anzeige und Ermittlung der Verunreinigungen mittels Ultraschall aus einem Gußerzeugnis oder einem bereits verformten Erzeugnis ein Probenrohling mit einer mindestens 10fachen Dicke der für die Ultraschalluntersuchung geeigneten Probendicke ausgeschnitten wird, die Dickenerstreckung des Probenrohlings in einer Ebene parallel zur Gießachse bzw. der Hauptverformungsrichtung des verformten Erzeugnisses liegt und planparallele Schnittflächen senkrecht zur Dickenerstreckung aufweist, daß der Probenrohling auf möglichst hohe Verformungstempertaur erwärmt und quer zur Dickenerstreckung auf die geeignete Probendicke verformt wird.

2. Verfahren nach Anspruch 1, dadurch gekennzeichnet, daß der Probenrohling mit mindestens einer Markierung längs seiner Dickenerstreckung versehen wird.

3. Verfahren nach den Ansprüchen 1 und 2, dadurch gekennzeichnet, daß bei einem Gußerzeugnis, das in einer Bogen-Stranggießanlage erzeugt wurde, die Markierung auf der der Gießunterseite entsprechenden Seite angebracht wird.

4. Verfahren nach den Ansprüchen 1 bis 3, dadurch gekennzeichnet, daß von den Proben Streifenförmige Untersuchungsproben an der der markierten Seite gegenüberliegenden Seite abgetrennt werden.

5. Verfahren nach den Ansprüchen 1 bis 4, dadurch gekennzeichnet, daß die Warmverformung durch Walzen mit folgenden Schritten erfolgt:

1. Walzen des Probenrohlings quer zu seiner Dickenerstreckung unter Beibehaltung dieser Walzrichtung auf etwa die Hälfte der Ausgangsdicke,

2. Drehen des Probenrohlings um 90° in der Walzebene,

3. Walzen des Probenrohlings unter Beibehaltung der Walzrichtung auf etwa 1/5 der Dicke bei Beginn dieses zweiten Walzvorganges und

4. Abtrennen von streifenförmigen Untersuchungsproben vom Probenblech.

6. Verfahren nach Anspruch 2, dadurch gekennzeichnet, daß das Walzen des Probenrohlings quer zur Dickenerstreckung in Richtung einer vertikalen Verbindungslinie von Gießunterseite zur Gießoberseite erfolgt.

7. Verfahren nach den Ansprüchen 1 bis 3, dadurch gekennzeichnet, daß die Verformung bei über 1100 °C, insbesondere bei etwa 1350 °C erfolgt.

8. Verfahren nach Anspruch 5, dadurch gekennzeichnet, daß bei Proben, die einem Erzeugnis mit runder Querschnittsform entnommen sind, die Untersuchungsproben am Scheitelbereich der großen Hauptachse abgetrennt werden.

## Claims

1. A method of producing specimens for determining impurities in metal and steel, in particular steel produced by a continuous casting process, characterised in that for displaying and determining the impurities by means of ultrasound, from a cast product or an already deformed product, a specimen blank, the thickness of which is at least 10 times the specimen thickness suitable for ultrasonic investigation, is cut out, the thickness dimension of the specimen blank extends in a plane parallel to the casting axis and the main deformation direction of the deformed product, and plane-parallel cut surfaces extend at right angles to the thickness dimension, that the specimen blank is heated to the highest possible deformation temperature and is deformed transversely to the thickness dimension, to the suitable specimen thickness.

2. A method as claimed in Claim 1, characterised in that the specimen blank is provided with at least one mark along its thickness dimension.

3. A method as claimed in Claims 1 and 2, characterised in that in the case of a cast product produced in a curved continuous casting apparatus, the mark is applied to the side corresponding to the casting underside.

4. A method as claimed in Claims 1 to 3, characterised in that strip-like test samples are separated from the specimens on the side opposite to the marked side.

5. A method as claimed in Claims 1 to 4, characterised in that the thermal deformation (hot working) is carried out by rolling in the following steps:
   1) rolling of the specimen blank transversely to its thickness dimension, retaining this rolling direction to approximately half the starting thickness,
   2) rotation of the specimen blank by 90° in the rolling plane,
   3) rolling of the specimen blank, retaining the rolling direction to approximately 1/5 of the thickness at the beginning of this second rolling process and
   4) separation of the strip shaped test samples from the specimen sheet metal.

6. A method as claimed in Claim 2, characterised in that the rolling of the specimen blank takes place transversely to the thickness dimension in the direction of a vertical connecting line from the casting underside to the casting upperside.

7. A method as claimed in Claims 1 to 3, characterised in that the deformation is carried out at a temperature exceeding 1100°C, in particular at approximately 1350°C.

8. A method as claimed in Claim 5, characterised in that in the case of specimens taken from a product of round cross-sectional formation, the test samples are separated from the uppermost region of the large main axis.

## Revendications

1. Procédé pour fabriquer des éprouvettes pour la détection d'impuretés dans des métaux et aciers, en particulier des aciers coulés par un procédé de coulée continue, caractérisé en ce que, pour la détection et la visualisation des impuretés au moyen d'ultrasons à partir d'un produit de coulée ou d'un produit déjà façonné, on découpe une ébauche d'éprouvette ayant une épaisseur égale à au moins dix fois l'épaisseur de l'éprouvette appropriée pour l'analyse par ultrasons, l'étendue de l'épaisseur de l'ébauche d'éprouvette se trouvant dans un plan parallèle à l'axe de coulée ou à la direction de déformation principale du produit façonné et présentant des surfaces de coupe à faces planes et parallèles perpendiculairement à l'étendue de l'épaisseur, en ce que l'on réchauffe l'ébauche d'éprouvette à la température de déformation la plus élevée possible et on la déforme transversalement à l'étendue de l'épaisseur à l'épaisseur appropriée de l'éprouvette.

2. Procédé selon la revendication 1, caractérisé en ce que l'ébauche d'éprouvette est munie d'au moins un repère le long de l'étendue de son épaisseur.

3. Procédé selon les revendications 1 et 2, caractérisé en ce que, pour un produit de coulée, qui a été fabriqué dans une installation de coulée continue, le repère est prévu sur le côté correspondant au dessous de coulée.

4. Procédé selon les revendications 1 à 3, caractérisé en ce que des éprouvettes d'analyse en forme de bande sont séparées des éprouvettes, sur le côté oposé au côté repéré.

5. Procédé selon les revendications 1 à 4, caractérisé en ce que la déformation à chaud a lieu par laminage avec les étapes suivantes :
   1 - laminage de l'ébauche d'éprouvette transversalement à l'étendue de son épaisseur en maintenant cette direction de laminage sur environ la moitié de l'épaisseur initiale,
   2 - rotation de l'ébauche d'éprouvette de 90° dans le plan de laminage,
   3 - laminage de l'ébauche d'éprouvette en maintenant la direction de laminage sur environ 1/5 de l'épaisseur au début de cette seconde phase de laminage, et

4 - séparation d'éprouvettes d'analyse en forme de bande de la tôle d'éprouvette.

6. Procédé selon la revendication 2, caractérisé en ce que le laminage de l'ébauche d'éprouvette a lieu transversalement à l'étendue de son épaisseur en direction d'une ligne de jonction verticale entre le dessous de coulée et le dessus de coulée.

7. Procédé selon les revendications 1 à 3, caractérisé en ce que la déformation a lieu à plus de 1100°C, en particulier à environ 1350°C.

8. Procédé selon la revendication 5, caractérisé en ce que, pour des éprouvettes qui sont prélevées d'un produit à section transversale ronde, les éprouvettes d'analyse sont séparées au niveau de la zone de sommet du grand axe principal.

# Fig.1

WR II

WR II  Oberseite

1

WR I

Dmr. 175mm

875 mm

Streckung 5 fach

WR I

350 mm

Breitung 2 fach

F i g. 2

Fig.3

Fig.4

# Fig.5

Original 160:1

WR II

WR I

0 10mm

Fig.6

# Fig.7

OS

Knüppel

US

OS

Vorblock, Bloom

horizontal ← → vertikal

US

OS

Rund

US

Bramme

OS

Schmalseite

US

OS = Oberseite, US = Unterseite

# Fig.8

vor Walzung — absägen

OS — US

Stauchung horizontal

OS=Oberseite — US=Unterseite

OS — US

20mm

Fig.9

Fig.10

Brommenoberseite

F i g. 11

1/2 Brommendicke

Brommenmitte

Fig.12

Brammenoberseite

1/2 Brammendicke

Schmalseite

Abstand in mm

Brammenmitte

-150    -100    -50    0    50    100    150